# EUROPÄISCHE PATENTSCHRIFT

(11) **EP 3 825 735 B1**
(45) Veröffentlichungstag und Bekanntmachung des Hinweises auf die Patenterteilung: **15.04.2026**
(21) Anmeldenummer: 19210601.1
(22) Anmeldetag: 21.11.2019
(51) Int. Cl.: G01T 1/178

(54) **ALPHASTRAHLENDETEKTOR MIT EINEM OPTISCHEN SENSOR ZUM MESSEN DER RADON-KONZENTRATION IN DER UMGEBUNGSLUFT**
ALPHA RAY DETECTOR WITH AN OPTICAL SENSOR FOR MEASURING RADON CONCENTRATION IN AMBIENT AIR
DÉTECTEUR DU RAYONNEMENT ALPHA DOTÉ D'UN CAPTEUR OPTIQUE PERMETTANT DE MESURER LA CONCENTRATION DE RADON DANS L'AIR AMBIANT

(43) Veröffentlichungstag der Anmeldung: 26.05.2021
(73) Patentinhaber: LivAir GmbH, 80992 München (DE)
(72) Erfinder: Waltl, Rudolf, 89426 Wittislingen (DE)
(74) Vertreter: Keilitz, Wolfgang

(56) Entgegenhaltungen:
- CN-U- 202 362 462
- KR-A- 20170 025 395
- KR-B1- 101 771 476
- US-A- 5 489 780
- YAMAMOTO S ET AL: "Development of a continuous radon concentration monitoring system in underground soil", NUCLEAR SCIENCE SYMPOSIUM CONFERENCE RECORD, 2000 IEEE LYON, FRANCE 15-20 OCT. 2000, PISCATAWAY, NJ, USA,IEEE, US, vol. 1, 15 October 2000 (2000-10-15), pages 6/313 - 6/316, XP010556576, ISBN: 978-0-7803-6503-2, DOI: 10.1109/NSSMIC.2000.949228
- MCDONALD E WRENN ET AL: "DESIGN OF A CONTINUOUS DIGITAL-OUTPUT ENVIRONMENTAL RADON MONITOR", IEEE TRANSACTIONS ON NUCLEAR SCIENCE,, vol. NS-22, no. 1, 1 February 1975 (1975-02-01), pages 645 - 648, XP001446806

## Beschreibung

Die Erfindung betrifft einen Alphastrahlendetektor mit einem optischen Sensor zum Messen der Radon-Konzentration in der Umgebungsluft.

### HINTERGRUND DER ERFINDUNG

Radon ist ein radioaktives Edelgas, das als Zwischenprodukt beim radioaktiven Zerfall von Radium, Actinium oder Thorium entsteht. Es kommt auf natürliche Weise in bestimmten Gesteinsschichten vor, von wo es bis zur Erdoberfläche gelangen kann. An der Erdoberfläche sammelt es sich bevorzugt in den Kellern von Gebäuden, wo es mitunter hohe Konzentrationen erreicht. Alle bekannten Isotope von Radon sind radioaktiv und strahlen beim Zerfall Alphateilchen (oder Betateilchen) aus. Da Radon und seine Folgeprodukte (Polonium, Blei und Wismut) vom Menschen zusammen mit der Umgebungsluft eingeatmet wird, ist es potentiell gesundheitsgefährdend.

Zur Messung der Radonkonzentration in der Umgebungsluft gibt es verschiedene Ansätze. Einer davon nutzt die Eigenschaft von Szintillatoren, also Materialien, die bei Auftreffen eines Alphateilchens einen Lichtimpuls aussenden. Die so erzeugten Lichtimpulse werden dann von einem optischen Sensor erfasst. Die Anzahl sowie die Stärke/Intensität der Lichtimpulse pro Zeiteinheit ist dabei ein Maß für die Radonkonzentration in der Umgebungsluft. Die aus dem Stand der Technik bekannten Radondetektoren mit einem optischen Sensor sind allesamt relativ aufwändig konstruiert, teuer, verbrauchen viel Energie und sind sehr groß, oder nicht ausreichend empfindlich oder träge.

Aus der CN 202 362 462 U, der KR 2017 002 539 5 A oder der US 5 489 780 A sind verschiedene Alphastrahlendetektoren zur Messung der Radonkonzentration in der Umgebungsluft bekannt. Weitere Alphastrahlendetektoren sind in den Veröffentlichungen: MCDONALD E. WRENN ET AL.: "DESIGN OF A CONTINUOUS DIGITAL-OUTPUT ENVIRONMENTAL RADON MONITOR", IEEE TRANSACTIONS ON NUCLEAR SCIENCE, 1. Februar 1975, Seiten 645 - 648 sowie YAMAMOTO S. ET AL.: "NUCLEAR SCIENCE SYMPOSIUM CONFERENCE RECORD", 2000, IEEE, Artikel "Development of a continuous radon concentration monitoring system in underground soil", Seiten: 6,313 - 6,316 beschrieben. Die aus dem Stand der Technik bekannten Alphastrahlendetektoren sind jedoch relativ komplex aufgebaut und aufwändig in ihrer Herstellung. Die KR 101 771 476 B1 beschreibt ferner einen Radondetektor mit einem Gehäuse, das einen Durchgang aufweist, durch den Umgebungsluft in den Innenraum des Gehäuses eindringen kann.

### AUFGABE DER ERFINDUNG

Es ist daher eine Aufgabe der vorliegenden Erfindung, einen Alphastrahlendetektor mit einem optischen Sensor zu schaffen, der wesentlich einfacher aufgebaut, sehr klein, sehr energiesparend, sehr empfindlich, schnell und kostengünstiger herzustellen ist.

Gelöst wird diese Aufgabe gemäß der Erfindung durch die im Patentanspruch 1 angegebenen Merkmale. Weitere Ausgestaltungen der Erfindung sind Gegenstand von Unteransprüchen.

Gemäß der Erfindung wird ein Alphastrahlendetektor mit einem optischen Sensor zum Messen der Radon-Konzentration in der Umgebungsluft vorgeschlagen, der gemäß Anspruch 1 ausgebildet ist. Ein solcher Alphastrahlendetektor ist sehr einfach aufgebaut und kann sehr klein, energiesparend und kostengünstig hergestellt werden.

Das Gehäuse und somit auch die Mess-Kammer des erfindungsgemäßen Radondetektors ist vorzugsweise vollständig geschlossen, hat also keine freie Öffnung. Um dennoch das Eindringen von Umgebungsluft in die Kammer zu ermöglichen, ist bei dem in Anspruch 1 definierten Alphastrahlendetektor ein ringförmiger Bereich der Haube aus einem Material hergestellt, das lichtundurchlässig aber luftdurchlässig/gasdurchlässig ist. Der in Anspruch 11 definierte Alphastrahlendetektor umfasst alternativ eine Basis mit einem Durchgang, der von dem lichtundurchlässigen aber gasdurchlässigen Material verschlossen ist. Die Materialstärke, der Aufbau und die Dichtigkeit des genannten Materials ist dabei so gewählt, dass kein Licht, aber ausreichend Umgebungsluft in die Kammer gelangen kann. Des Weiteren ist das Material vorzugsweise so gewählt, dass bereits zerfallenes Radon, also Polonium, Blei oder Wismut nicht von außen in die Kammer eindringen kann. Da diese Folgezerfälle von Radon ionisiert sind, werden diese durch das Material gebunden und können somit nicht in die Kammer eindringen.

Bei dem genannten gasdurchlässigen Material der Gehäusewand kann es sich beispielsweise um Filz, Silikon, Stoff, einen Kunststoff oder um eine Membran, oder um ein anderes Material mit den genannten Eigenschaften handeln.

Gemäß der Erfindung wird außerdem eine zweite Ausführungsform eines Alphastrahlendetektors vorgeschlagen, wie sie in Anspruch 11 definiert ist. Der Alphastrahlendetektor umfasst in diesem Fall eine Basis mit einem Durchgang, der von einem geeigneten Material lichtdicht, aber gasdurchlässig verschlossen wird. Das genannte Material kann wahlweise innerhalb des Durchgangs, an der Innenseite der Gehäusewand und/oder an der Außenseite der Gehäusewand angebracht sein, so dass der Durchgang verschlossen ist.

Beide Ausführungsformen des hier vorgeschlagenen Alphastrahlendetektors haben eine Haube mit einer Innenwand, die mit einem Szintillationsmaterial versehen ist. Bei dem genannten Szintillationsmaterial kann es sich beispielsweise um Zinksulfid, Bismutgermanat, Bleiwolframat, Lutetiumoxyorthosilicat, Natriumiodid, Zinksulfid oder Caesiumiodid handeln.

Das Szintillationsmaterial ist vorzugsweise eine Schicht, die an der Innenwand der Kammer aufgetragen ist.

Beide Ausführungsformen des erfindungsgemäßen Alphastrahlendetektors umfassen eine Basis, auf der eine Haube angeordnet ist, die z.B. die Form einer Kuppel haben kann. Die eingangs genannte Kammer befindet sich innerhalb der Haube. Bei der ersten Ausführungsform gemäß Anspruch 1 ist der optische Sensor vorzugsweise innerhalb der Kammer und vorzugsweise auf der Basis angeordnet.

Die Haube kann beispielsweise als Teil einer Sphäre ausgebildet sein. Sie kann z. B. halbkugelförmig oder pyramidenförmig gebildet sein. Sie kann aber auch zylindrisch oder eckig, z. B. quaderförmig, ausgebildet sein.

Bei dem optischen Sensor des Alphastrahlendetektors gemäß Anspruch 1 handelt es sich vorzugsweise um einen Photomultiplier, insbesondere einen SiPM. Der optische Sensor ist vorzugsweise innerhalb der Mess-Kammer angeordnet.

Die Basis des Alphastrahlendetektors gemäß Anspruch 1 kann beispielsweise plattenförmig ausgebildet sein. Sie besteht vorzugsweise aus einem Leiterplattenmaterial. Dies hat den Vorteil, dass die Auswerteelektronik gleich auf oder unter der Basis angeordnet werden kann.

### KURZE BESCHREIBUNG DER ZEICHNUNGEN

Die Erfindung wird nachstehend anhand der beigefügten Zeichnung beispielhaft näher erläutert. Es zeigen:

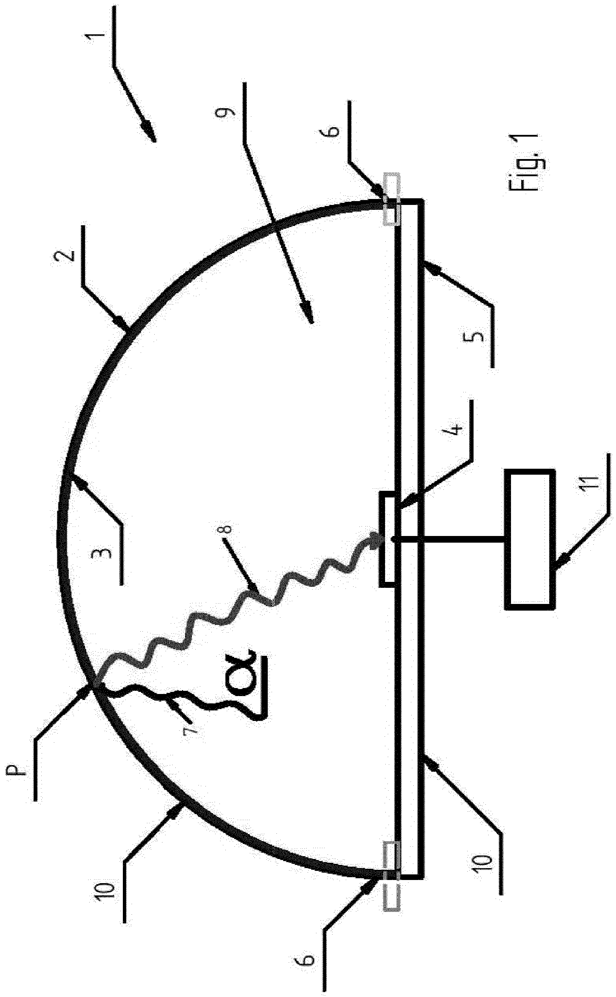

Fig. 1 zeigt einen Alphastrahlen- bzw. Radondetektor 1 mit einem mehrteiligen Gehäuse 10, das eine Basis 5 umfasst, auf der eine halbkugelförmige Haube 2 angeordnet ist. Innerhalb der Haube 2 befindet sich eine Kammer 9, welche eine Messzelle darstellt, in der die Radonkonzentration gemessen wird.

Das Gehäuse 10 des Radondetektors 1 ist vollständig geschlossen, so dass kein Licht von außen in die Kammer 9 eindringen kann. Ein ringförmiger Bereich 6 der Haube 2 ist allerdings aus einem Material hergestellt, das lichtundurchlässig, aber luftdurchlässig / gasdurchlässig / radondurchlässig ist, so dass Umgebungsluft von außen in die Kammer 9 eindringen kann.

Das luftdurchlässige Material kann beispielsweise Filz, Silikon, Stoff, einen Kunststoff oder eine Membran umfassen. Die Wandstärke und der Aufbau / die Dichtigkeit des Materials ist derart gewählt, dass es für das am Einsatzort herrschende Licht undurchlässig ist, aber ein ausreichender Austausch von Umgebungsluft innerhalb der Kammer stattfinden kann. Die übrige Haube 2 kann z. B. aus Metall oder einem Kunststoff hergestellt sein.

An der Innenwand der Haube 2 und ggf. auch auf der Basis 5 ist ein Szintillationsmaterial 3 vorgesehen, das bei Auftreffen von Alphastrahlung Lichtimpulse erzeugt, die dann vom optischen Sensor 4 erfasst werden. Wie eingangs erwähnt wurde, zerfällt Radon unter Ausstrahlung von Alphateilchen in weitere Zerfallsprodukte. Auch die Zerfallsprodukte wie z.B. Polonium zerfällt dann erneut mit Alphastrahlung. Ein Alphateilchen 7 ist in Fig. 1 exemplarisch dargestellt. Das Alphateilchen 7 bewegt sich in Richtung des Pfeils und stößt an einem Punkt P gegen das an der Innenwand der Haube 2 befindliche Szintillationsmaterial 3. Dieses sendet wiederum einen optischen Lichtimpuls 8 aus, der dann vom optischen Sensor 4 detektiert wird. Bei dem Szintillationsmaterial 3 handelt es sich vorzugsweise um eine Schicht aus Zinksulfid (ZnS:Cu, ZnS:Ag). Alternativ könnten auch andere aus dem Stand der Technik bekannte Materialien verwendet werden.

Die Basis 5 umfasst eine Leiterplatte, auf oder unter der auch eine Auswerteelektronik 11 angeordnet werden kann. Bei dem optischen Sensor 4 handelt es sich vorzugsweise um einen Silizium-Photomultiplier. Im dargestellten Ausführungsbeispiel sitzt der optische Sensor 4 am Boden der Kammer 9 auf der Basis 5.

Eine Optik zur Bündelung der Lichtimpulse 8 kann, muss aber nicht vorgesehen sein.

Die gemessene Radonkonzentration wird vorzugsweise auf einem Display (nicht gezeigt) angezeigt. Der Radondetektor 1 kann auch eine oder mehrere Schnittstellen für Peripheriegeräte, wie z.B. Anzeigeeinheiten oder Computer, aufweisen.

## Patentansprüche

1. Alphastrahlendetektor (1) zum Messen der Radon-Konzentration in der Umgebungsluft, umfassend:
- ein mehrteilig ausgebildetes Gehäuse (10) mit einer Basis (5), auf der eine Haube (2) angeordnet ist, in der sich eine Kammer (9) befindet, wobei das Gehäuse (10) derart ausgeführt ist, dass von außen Umgebungsluft in die Kammer (9) eindringen kann, und
- einen optischen Sensor (4);
wobei die Haube (2) eine Innenwand aufweist, die mit einem Szintillationsmaterial (3) versehen ist, das bei Auftreffen von Alphateilchen (7) Lichtimpulse (8) erzeugt, die vom optischen Sensor (4) erfasst werden; **dadurch gekennzeichnet, dass**
die Haube (2) ferner einen ringförmigen Bereich (6) aufweist, der aus einem Material hergestellt ist, das lichtundurchlässig aber luftdurchlässig ist, so dass Umgebungsluft von außen in die Kammer (9) eindringen kann.

2. Alphastrahlendetektor (1) nach Anspruch 1,
**dadurch gekennzeichnet, dass**
das Gehäuse (10) eine Gehäusewand hat, die die Kammer (9) vollständig umschließt.

3. Alphastrahlendetektor (1) nach Anspruch 1,
**dadurch gekennzeichnet, dass**
das luftdurchlässige Material Filz, Silikon, Stoff, Kunststoff oder eine Membran umfasst.

4. Alphastrahlendetektor (1) nach einem der vorhergehenden Ansprüche,
**dadurch gekennzeichnet, dass**
das Szintillationsmaterial (3) Zinksulfid, Bismutgermanat, Bleiwolframat, Lutetiumoxiorthosilicat, Natriumiodid oder Caesiumiodid umfasst.

5. Alphastrahlendetektor (1) nach Anspruch 1,
**dadurch gekennzeichnet, dass**
der optische Sensor (4) auf der Basis (5) innerhalb der Kammer (9) angeordnet ist.

6. Alphastrahlendetektor (1) nach Anspruch 1,
**dadurch gekennzeichnet, dass**
die Haube (2) ein Teil einer Sphäre ist.

7. Alphastrahlendetektor (1) nach einem der vorhergehenden Ansprüche,
**dadurch gekennzeichnet, dass**
der optische Sensor (4) ein Fotomultiplier ist.

8. Alphastrahlendetektor (1) nach einem der vorhergehenden Ansprüche,
**dadurch gekennzeichnet, dass**
der optische Sensor (4) in der Kammer (9) angeordnet ist.

9. Alphastrahlendetektor (1) nach Anspruch 1,
**dadurch gekennzeichnet, dass**
die Basis (5) plattenförmig ausgebildet ist.

10. Alphastrahlendetektor (1) nach Anspruch 1,
**dadurch gekennzeichnet, dass**
die Basis (5) aus einem Leiterplattenmaterial hergestellt ist.

11. Alphastrahlendetektor (1) zum Messen der Radon-Konzentration in der Umgebungsluft, umfassend
- ein mehrteilig ausgebildetes Gehäuse (10) mit einer Basis (5), auf der eine Haube (2) angeordnet ist, in der sich eine Kammer (9) befindet; wobei das Gehäuse (10) derart ausgeführt ist, dass von außen Umgebungsluft in die Kammer (9) eindringen kann, und
- einen optischen Sensor (4);
wobei die Haube (2) eine Innenwand aufweist, die mit einem Szintillationsmaterial (3) versehen ist, das bei Auftreffen von Alphateilchen (7) Lichtimpulse (8) erzeugt, die vom optischen Sensor (4) erfasst werden; **dadurch gekennzeichnet, dass**
- die Basis (5) aus einem Leiterplattenmaterial hergestellt ist;
- eine Auswerteelektronik (21) auf oder unter der Basis (5) angeordnet ist;
- der optische Sensor (4) auf der Basis (5) innerhalb der Kammer (9) angeordnet ist;
- der optische Sensor (4) ein Silizium-Photomultiplier ist; und
- die Basis (5) einen Durchgang aufweist, der von einem lichtundurchlässigen aber gasdurchlässigen Material (6) verschlossen ist, so dass Radongas von außen, durch den Durchgang (16) in die Kammer (9) eindringen kann.

## Claims

1. Alpha radiation detector (1) for measuring the radon concentration in the ambient air, comprising:
- a multi-part housing (10) with a base (5) on which a cover (2) is arranged, in which a chamber (9) is located, wherein the housing (10) is designed such that ambient air can enter the chamber (9) from the outside, and
- an optical sensor (4);
wherein the hood (2) has an inner wall provided with a scintillation material (3) which, upon impact with alpha particles (7), generates light pulses (8) that are detected by the optical sensor (4);
**characterised in that**
the hood (2) further comprises an annular region (6) made of a material that is opaque to light but permeable to air, so that ambient air can enter the chamber (9) from the outside.

2. Alpha radiation detector (1) according to claim 1,
**characterised in that**
the housing (10) has a housing wall that completely encloses the chamber (9).

3. Alpha radiation detector (1) according to claim 1,
**characterised in that**
the air-permeable material comprises felt, silicone, fabric, plastic or a membrane.

4. Alpha radiation detector (1) according to one of the preceding claims,
**characterised in that**
the scintillation material (3) comprises zinc sulphide, bismuth germanate, lead tungstate, lutetium oxyorthosilicate, sodium iodide or caesium iodide.

5. Alpha ray detector (1) according to claim 1,
**characterised in that**
the optical sensor (4) is arranged on the base (5) inside the chamber (9).

6. Alpha radiation detector (1) according to claim 1,
**characterised in that**
the cover (2) is part of a sphere.

7. Alpha radiation detector (1) according to one of the preceding claims,
**characterised in that**
the optical sensor (4) is a photomultiplier.

8. Alpha radiation detector (1) according to one of the preceding claims,
**characterised in that**
the optical sensor (4) is arranged in the chamber (9).

9. Alpha radiation detector (1) according to claim 1,
**characterised in that**
the base (5) is designed in the form of a plate.

10. Alpha radiation detector (1) according to claim 1,
**characterised in that**
the base (5) is made of a printed circuit board material.

11. Alpha radiation detector (1) for measuring the radon concentration in the ambient air, comprising
- a multi-part housing (10) with a base (5) on which a cover (2) is arranged, in which a chamber (9) is located; wherein the housing (10) is designed such that ambient air can enter the chamber (9) from the outside, and
- an optical sensor (4);
wherein the hood (2) has an inner wall provided with a scintillation material (3) which, upon impact with alpha particles (7), generates light pulses (8) that are detected by the optical sensor (4);
**characterised in that**
- the base (5) is made of a printed circuit board material ;
- evaluation electronics (21) are arranged on or under the base (5);
- the optical sensor (4) is arranged on the base (5) inside the chamber (9)
- the optical sensor (4) is a silicon photomultiplier; and
- the base (5) has a passageway that is closed by an opaque but gas-permeable material (6), so that radon gas can enter the chamber (9) from the outside through the passageway (16) .

## Revendications

1. Détecteur de rayonnement alpha (1) pour mesurer la concentration de radon dans l'air ambiant, comprenant:
- un boîtier (10) composé de plusieurs parties, avec une base (5) sur laquelle est disposé un capot (2) dans lequel se trouve une chambre (9), le boîtier (10) étant conçu de telle sorte que l'air ambiant puisse pénétrer dans la chambre (9) depuis l'extérieur, et
- un capteur optique (4);
le capot (2) présentant une paroi intérieure pourvue d'un matériau scintillant (3) qui, lorsqu'il est frappé par des particules alpha (7), génère des impulsions lumineuses (8) qui sont détectées par le capteur optique (4);
**caractérisé en ce que**
le capot (2) comporte en outre une zone annulaire (6) réalisée dans un matériau qui est opaque à la lumière mais perméable à l'air , de sorte que l'air ambiant peut pénétrer de l'extérieur dans la chambre (9).

2. Détecteur de rayonnement alpha (1) selon la revendication 1,
**caractérisé en ce que**
le boîtier (10) comporte une paroi qui entoure complètement la chambre (9).

3. Détecteur de rayonnement alpha (1) selon la revendication 1,
**caractérisé en ce que**
le matériau perméable à l'air comprend du feutre, du silicone, du tissu, du plastique ou une membrane.

4. Détecteur de rayonnement alpha (1) selon l'une des revendications précédentes,
**caractérisé en ce que**
le matériau scintillateur (3) comprend du sulfure de zinc, du germanate de bismuth, du tungstate de plomb, de l'orthosilicate de lutétium, de l'iodure de sodium ou de l'iodure de césium.

5. Détecteur de rayonnement alpha (1) selon la revendication 1,
**caractérisé en ce que**
le capteur optique (4) est disposé sur la base (5) à l'intérieur de la chambre (9).

6. Détecteur de rayonnement alpha (1) selon la revendication 1,
**caractérisé en ce que**
le capot (2) fait partie d'une sphère.

7. Détecteur de rayonnement alpha (1) selon l'une des revendications précédentes,
**caractérisé en ce que**
le capteur optique (4) est un photomultiplicateur.

8. Détecteur de rayonnement alpha (1) selon l'une des revendications précédentes,
**caractérisé en ce que**
le capteur optique (4) est disposé dans la chambre (9).

9. Détecteur de rayonnement alpha (1) selon la revendication 1,
**caractérisé en ce que**
la base (5) est conçue en forme de plaque.

10. Détecteur de rayonnement alpha (1) selon la revendication 1,
**caractérisé en ce que**
la base (5) est fabriquée à partir d'un matériau de carte de circuit imprimé.

11. Détecteur de rayonnement alpha (1) pour mesurer la concentration de radon dans l'air ambiant, comprenant
- un boîtier (10) formé de plusieurs parties avec une base (5) sur laquelle est disposé un capot (2) dans lequel se trouve une chambre (9); le boîtier (10) étant conçu de telle sorte que l'air ambiant puisse pénétrer dans la chambre (9) depuis l'extérieur, et
- un capteur optique (4);
le capot (2) présentant une paroi intérieure pourvue d'un matériau scintillant (3) qui, lorsqu'il est frappé par des particules alpha (7), génère des impulsions lumineuses (8) qui sont détectées par le capteur optique (4) ; **caractérisé en ce que**
- la base (5) est fabriquée à partir d'un matériau de carte de circuit imprimé;
- un circuit électronique d'évaluation (21) est disposé sur ou sous la base (5);
- le capteur optique (4) est disposé sur la base (5) à l'intérieur de la chambre (9);
- le capteur optique (4) est un photomultiplicateur à silicium; et
- la base (5) comporte un passage qui est fermé par un matériau opaque mais perméable aux gaz (6), de sorte que le radon provenant de l'extérieur peut pénétrer dans la chambre (9) par le passage (16).
